# EUROPEAN PATENT APPLICATION

(11) **EP 2 510 780 A1**
(43) Date of publication of application: **17.10.2012**
(21) Application number: 11159906.4
(22) Date of filing: 25.03.2011
(51) Int. Cl.: A01H 5/08

(54) **Novel squash plant for protected culture**

(71) Applicant: Syngenta Participations AG, 4058 Basel (CH)
(72) Inventor: Leibovitz, Gadi, 70750, Gedera (IL); Debreczeni, Erika, 70750, Gedera (IL)
(74) Representative: Bessiere, Philippe Jean Luc

(57) **Abstract**

The present invention relates to novel summer squash (Cucurbita pepo L.) plants displaying a novel growth habit and high level of parthenocarpic fruit set. The plants disclosed within the present invention provide the grower with novel solutions to enhance its economical and commercial efficiency when summer squash fruits are to be produced under protected conditions, and more or less independently of the climate and temperature. The invention discloses a cultivated Cucurbita pepo plant, which is capable of producing parthenocarpic fruits and exhibits elongated internode length, wherein the parthenocarpic trait and elongated internodes length trait are controlled by genetic factors and independent of pollination and parthenocarpy-inducing chemical treatments.

## Description

### FIELD OF THE INVENTION

The present invention relates to novel summer squash *(Cucurbita pepo* L.) plants displaying a novel growth habit and high level of parthenocarpic fruit set. The present invention also relates to seeds and parts of said plants, for example fruits. The present invention also relates to methods of making and using such seeds and plants.

### BACKGROUND OF THE INVENTION

Summer squash *(Cucurbita pepo* L.), which is ranking high in economic importance among vegetable crops worldwide, is renowned as one of the easiest vegetables to cultivate in temperate climate. However, this cultivation is easily challenged by a lack of available pollinators. Summer squash carries both male and female flowers on the same plant, and is pollinated by nectar-collecting bees. Thus in situations when the bee population is limited, during the winter, or when the plants are grown under protected conditions (as opposed to outdoors), producers often experience fruit abortion. The fruit begins to grow, then dries or rots. This is due to an insufficient number of pollen grains delivered to the female flower.

Since natural pollination is often very limited when using protected crop cultivation method, growers must induce normal fruit development by applying different growth regulators on plants once a week or every morning directly on stigmata into open female flowers. A significant positive effect of growth regulators on yield and earliness in zucchini summer squash grown in protected cultivation was reported by Mancini *et al.* and Suleiman *et al.* However, the known chemicals could have multiple undesired and deleterious effects on the plants and the environment. The implementation is laborious, and sometimes the effectiveness is unpredictable (Suleiman *et al.*, 1990).

An alternative and natural way to obtain fruits in conditions where pollination is limited or does not occur is by using varieties that perform parthenocarpy. Parthenocarpy in summer squash is defined as fruit set where the mating stage (pollination of stigmata) is missing, and thus the fruits are seedless. In addition to its useful contribution to the safety of squash supply during adverse conditions, parthenocarpy participates in fruit quality improvement thanks to an increase of dry matter as well as absence of any chemical residues.

Besides its susceptibility to temperature and daylight, parthenocarpy as defined above is driven by genetic factors. Robinson R.W. and Reiners S. compared 36 summer squash cultivars for their ability to set parthenocarpic fruits in the field. Some cultivars set no parthenocarpic fruit, and others varied in the amount of fruit set when not pollinated. They concluded that parthenocarpy can supplement insect pollination and improve fruit set of squash when conditions are unfavorable for pollination, but the cultivars tested cannot be expected to produce normal yield by parthenocarpy alone. None of the cultivars or breeding lines tested set as many fruit by parthenocarpy as when insect-pollinated, and in any case, the quality of fruit obtained was below acceptance for commercial cultivars. In most of the cases, parthenocarpy is fortuitous and fruits are shrunken and deformed. For example, Nijs *et al.*, 1982 screened a collection of 19 summer squash cultivars for parthenocarpic fruit development when grown at low night temperature in an insect-proof Venlo-type glasshouse in Wageningen, Netherlands. Indeed, only three cultivars yielded three fruits or more per plant or above 1000 grams per plant. The percentage of first quality fruit was not correlated with the degree of parthenocarpy. Cultivar designated as DG-4 which is a progeny of cultivar Dark Green Zucchini (by Otis Twilley Seeds Co) presented higher yield compared to other varieties. A continued study presented preliminary evidence that the high level of parthenocarpy in DG-4 is a recessively inherited character. The hybrid summer squash that carries parthenocarpic trait was presented in US Patent 6031158, which disclosed a squash plant of the species *Cucurbita pepo* selected from a population of progeny of the hybrid produced by crossing and DG4 and Striata having an inherited trait for parthenocarpy, or parts thereof. DG4 is Dutch public release (Nijs *et al.* 1982), the other being a public variety known as "Striata," "Striato" or "Striata d'Italia" which is well adapted to the environment of Italy. DG4 is a very compact, bushy plant with dark leaves and very dark ("black") short fruits. According to the "official" (PVP Office) description of the variety, the mature hybrid plant is of the glabrous bush type, with an average of 15 internodes on a round main stem of 28 cm long.

Such parthenocarpic squash plants are therefore not only unmarketable due to highly reduced yields but also absolutely not adapted for growing under protected conditions. Indeed, the summer squash commercial varieties are mainly bred for outdoor production, yielding a preferred phenotype of a bushy and highly vigorous plant. These traits make the plants unsuitable for indoor, protected cultivation due to the fact that it is nearly impossible to implement growing methods such as trellising and plant support techniques used successfully in other protected vegetable production, such as with tomatoes and cucumbers. Additionally, the management of squash plants under protected conditions is usually laborious when compared with the advantages seen with other protected vegetable cultivation. Thus there is an unmet need for squash plants adapted for cultivation in a protected area.

The growing of summer squash out-of-season in protected cultivation is of interest (plastic houses, glasshouses or net houses) for its high yields and good commercial value. Planting summer squash in plastic protected greenhouses has special economical value when planted during the late summer in order to provide the product during winter time, then securing squash supply during non-favorable conditions. Furthermore, protected conditions, such as greenhouses, present several advantages with respect to field production. In greenhouses, crops exhibit greater yields, higher quality, and the harvest season is extended per unit of cropped area. High-value crops can be grown in soil-less media and irrigated with water and nutrients which can be recycled into the cropping system. Pests and diseases can be managed using biological control strategies. The high returns commanded by specialty crops grown in greenhouses can make this production system a viable alternative for growers in regions where production constraints such as unfavorable climate, reduced land due to urbanization, and restriction and reduction in the use of pesticides make field production more difficult (Luzny J.).

Thus, there remains a long felt and unmet need for a more effective Zucchini squash phenotype displaying a higher adaptability to cultivation under protected conditions. In particular, zucchini squash plants exhibiting a high yield of parthenocarpic marketable fruits and new growth habits adapted for growing in a protected area would undoubtedly improve the range of possibilities given to a grower for producing squash fruits under adverse conditions.

### SUMMARY OF THE INVENTION

The present invention addresses the need for more constant supply of *Cucurbita pepo* fruits along the year. To solve this problem, the present invention provides novel Zucchini squash plants that display an increased adaptability to cultivation under protected area. In particular, the invention discloses novel Zucchini squash plants capable of producing parthenocarpic marketable and high-quality fruits without pollination, nor chemical treatments, thus improving the grower capability to make profit of a regular production of summer squash all year long. More particularly, the invention discloses novel Zucchini squash plants adapted to growth under protected conditions, characterized in that they display particular genetic traits including an elongated internode length trait and a shorter petioles trait. These phenotypic features widely facilitate the work of the grower towards squash production and harvest, especially by facilitating fruits accessibility within the squash plant. Furthermore, the novel squash plant herein disclosed allows higher yield since it can set much more fruits per square meter for a given area.

Altogether, the characteristics of the plants disclosed within the present invention provide the grower with novel solutions to enhance its economical and commercial efficiency when summer squash fruits are to be produced under protected conditions, and more or less independently of the climate and temperature.

In embodiment 1, the invention discloses a cultivated *Cucurbita pepo* plant, which is capable of producing parthenocarpic fruits and exhibits elongated internode length, wherein the parthenocarpic trait is controlled by a genetic factor and is independent of pollination and parthenocarpy-inducing chemical treatments regimes, and wherein the elongated internode length trait is controlled by a genetic factor and is characterized in that the internode length is 1.5 to 5 times longer, particularly 2 to 4 times longer, more particularly 2 to 3.5 times longer than the internode length of a cultivated *Cucurbita pepo* plant of the same type at the same ready-to-harvest stage.

In embodiment 2, the invention discloses a plant according to embodiment 1, wherein said elongated internode length trait is characterized in that the internode length is comprised between 3.5 and 11 cm, preferably between 5 and 11 cm, but more preferably between 5 and 9 cm.

In embodiment 3, the invention discloses a plant according to any one of embodiments 1 to 2, wherein said parthenocarpic trait and said elongated internode length trait are obtainable from a donor plant which has the genetic background of *Cucurbita pepo* plant PARTYEL, representative seed of which is deposited under NCIMB Accession No. 41814.

In embodiment 4, the invention discloses a plant according to any one of embodiments 1 to 3, wherein said plant is an inbred, a dihaploid or a hybrid.

In embodiment 5, the invention discloses a plant according to any of the embodiments 1 to 4, obtainable by crossing *Cucurbita pepo* plant PARTYEL, representative seed of which is deposited under NCIMB Accession No. 41814, with any *Cucurbita pepo* plant that does not contain at least one trait selected in the group comprising parthenocarpic trait and elongated internode length trait.

In embodiment 6, the invention discloses seed of a *Cucurbita pepo* plant according to any one of embodiments 1 to 5.

In embodiment 7, the invention discloses fruit of a *Cucurbita pepo* plant according to any one of embodiments 1 to 5.

In embodiment 8, the invention discloses the use of a *Cucurbita pepo* cultivation material, seed or plant, comprising genetic factors contributing to parthenocarpic trait and elongated internode length trait for cultivation on a support in a protected area for production and harvesting of fruits, independently of any chemical treatment and/or without any pollination.

In embodiment 9, the invention discloses the use of a cultivated *Cucurbita pepo* plant material according to embodiment 8, wherein said material is a material according to any one of embodiments 1 to 6.

In embodiment 10, the invention discloses the use of a cultivated *Cucurbita pepo* plant, or plant part, according to any one of embodiments 1 to 6 to produce a hybrid *Cucurbita pepo* plant suitable for cultivation on a support in a protecting area for production and harvest of fruits.

In embodiment 11, the invention discloses a method for producing *Cucurbita pepo* fruits from a *Cucurbita pepo* plant without pollination, nor chemical treatment, wherein said method comprises steps of:
a) providing a *Cucurbita pepo* cultivation material, seed or plant, wherein said material is a material according to any one of embodiments 1 to 6,
b) growing the material provided in a protected area and,
c) directing the growth of the plant along a support or trellis until fruit setting, and,
d) harvesting the fruits once they reach the ready-to-harvest stage.

In embodiment 12, the invention discloses a method of providing a *Cucurbita pepo* material, seed or plant, wherein said method comprises the following steps:
a) Providing a 1^{st} *Cucurbita pepo* plant,
b) Crossing said 1^{st} plant with a 2^{nd} *Cucurbita pepo* plant comprising a "parthenocarpic" trait and a "elongated internode length" trait,
c) Obtaining a progeny *Cucurbita pepo* plant and,
d) Selecting a plant of said progeny characterized in that said plant produces parthenocarpic fruits in a protected area without pollination nor chemical treatment, and wherein said plant is characterized by an elongated internode length.

In embodiment 13, the invention discloses a method according to embodiment 12, wherein the 2^{nd} *Cucurbita pepo* plant is chosen in the group comprising plant according to any one of embodiments 1 to 5.

In embodiment 14, the invention discloses a cell of a plant according to any one of embodiments 1 to 5.

### DETAILED DESCRIPTION OF THE INVENTION

The present invention addresses the need for more constant supply of *Cucurbita pepo* fruits along the year. To solve this problem, the present invention provides novel Zucchini squash plants that display a increased adaptability to cultivation under protected area. In particular, the invention discloses novel Zucchini squash plants capable of producing parthenocarpic fruits without pollination, nor chemical treatments. More particularly, the invention discloses novel Zucchini squash plants adapted to growth under protected conditions, characterized in that they display particular genetic traits including an elongated internode length trait and a shorter petioles trait.

### DEFINITIONS:

The term "*Cucurbita pepo* plant" also refers to squash plants, summer squash plants as well as Zucchini plants, green or yellow.

'Stigmata' are herein defined as the receptive apex of the pistil of a flower, on which pollen is deposited at pollination.

'Parthenocarpy' or 'parthenocarpic" is herein defined as the ability for plant to produce seedless fruits which set where the mating stage (pollination of stigmata) is missing, thus the fruits are substantially seedless. Parthenocarpy is further defined as the production of fruit without pollination of ovules.

'Chemical treatments' as herein used refer to factors or agents that, upon exogenous application to the plant, particularly to the reproductive parts of the plant such as, for example, the stigmata, are capable of inducing an artificial parthenocarpy in the treated plant. Such exogenous factors may be, for example, dead pollen extract, growth regulating substances, either natural or synthetic such as plant hormones including auxins, gibberellins and cytokines, auxin transport inhibitors, or others.

'Protected conditions' or 'controlled conditions' herein refer to the conditions employed by growers of summer squash *(Cucurbita pepo* L.) for production in glasshouses, plastic houses net houses and similar structures and modes. Such structures and modes are adapted to prevent natural pollination insect activity of said summer squash plants.

The terms 'Protected area' or 'controlled area' herein refer to structures such as greenhouses, glasshouses, hot houses and any other structures employed by growers of summer squash *(Cucurbita pepo* L.) for production under the aforementioned 'Protected conditions' or'controlled conditions'

The term 'Open growth habit' herein refers to the plant structure related traits such as short leaf petioles, longer internodes and smaller leaf size characterizing the novel summer squash plants of the present invention. These traits are herein identified to increase the effectiveness of greenhouse space usage in summer squash production. Such an open growth habit reduces disease incidence and chemical sprays usage and enables the plant to be easily grown in higher density and thus increases the number of plants per square meter. The aforementioned advantages of the novel summer squash plants are adapted to the special climatic and technological conditions characterizing protected cultivation.

The term "support for growth" is meant to be understood as vertical, angled or horizontal support by means of a trellis, wire, rod, stick, cane or any other convenient means known in the art.

The term "genetic factor" is intended to be understood herein as meaning a heritable element of the plant genome that contributes to the phenotype and that can be transmitted to the plant's progeny. The term "genetic factor" further refers to a genetic locus which is stably incorporated into the genome of a plant and which confers on the plant a characteristic phenotype. A "genetic factor" is understood herein to refer to a heritable genetic element that is capable of contributing to the expression of a trait on the level of the DNA itself, on the level of translation, transcription and/or activation of a final polypeptide product, leading to the phenotypic expression of the trait. In one preferred embodiment, the genetic factors contributing to the traits of the plants according to the present invention are native to the *Cucurbita* genus. The genetic factors according to the present invention do not comprise genetically modified events comprising heterologous genetic material and/or genetic material foreign to *Cucurbita* genus.

The term "trait" refers to a characteristic or a phenotype. In the context of the present invention, a trait is for example a plant showing long internodes as described herein. A trait may be inherited in a dominant or recessive manner, or in a partial or incomplete-dominant manner. A trait may be monogenic or polygenic, or may result from the interaction of one or more genes with the environment.

The terms "hybrid", "hybrid plant", and "hybrid progeny" refer to an individual produced from genetically different parents (e.g. a genetically heterozygous or mostly heterozygous individual).

The term "inbred line" refers to a genetically homozygous or nearly homozygous population. An inbred line, for example, can be derived through several cycles of brother/sister breeding or of selfing or in dihaploid production. An "inbred", "inbred individual, or "inbred progeny" is an individual sampled from an inbred line.

The term "dihaploid line" refers to stable inbred lines issued from anther culture. Some pollen grains (haploid) cultivated on specific medium and circumstances can develop plantlets containing n chromosomes. These plantlets are then "double" and contain 2n chromosomes. The progeny of these plantlets are named "dihaploid" and are essentially not segregating any more (stable).

The term "pruning" is explained as follows: an arboricultural practice involving the selective removal of parts of a plant. This practice usually entails removal of diseased, damaged, dead, non-productive, or otherwise unwanted tissue from a plant, such as branches, buds, or roots. Reasons to prune plants include deadwood removal, shaping (by controlling or directing growth), improving or maintaining health, reducing risk from falling branches, preparing nursery specimens for transplant, and both harvesting and increasing the yield or quality of flowers and fruits.

The term "cultivar" or "variety" refers to a horticultural derived variety, as distinguished from a naturally occurring variety. In some embodiments of the present invention the cultivars or varieties are commercially valuable.

A "cultivated *Cucurbita pepo* plant" is understood within the scope of the invention to refer to a plant that is no longer in the natural state but has been developed and domesticated by human care and for agricultural use and/or human consumption. As a matter of example, *Cucurbita pepo* plant according to the present invention can be selected in the group comprising gem squash, summer squash, winter squash, zucchini, yellow crookneck squash, yellow summer squash, cocozelle, scallop, straightneck and vegetable marrow. In the context of the present invention, a "cultivated *Cucurbita pepo* plant" is adapted to cultivation and exhibits disease resistance, particularly Intermediate Resistance, such as Zucchini Yellow Mosaic Virus (ZYMV) Intermediate Resistance.

The term "genetically fixed" refers to a genetic factor which has been stably incorporated into the genome of a plant that normally does not contain the genetic factor. When genetically fixed, the genetic factor can be transmitted in a predictable manner to other plants by sexual crosses.

The term "plant" or "plant part' refers hereinafter to any plant, plant organ or tissue including without limitation, fruits, seeds, embryos, meristematic regions, callus tissue, flowers, leaves, roots, shoots, gametophytes, sporophytes pollen, and microspores.

The term "elongated internode length" is herein understood as a length between about 3.5 cm and about 11 cm, preferably between about 5 cm and about 11 cm, more preferably between about 8 cm and 11 cm. It is understood in the context of the present application that the elongated internode length is measured once a fruit reaches its marketable size and is therefore at the ready-to-harvest stage. The measurement concerns the internode immediately underneath the node for which a fruit has reached its marketable size. Relatively speaking, the term "elongated internode length" is herein understood to mean that the internodes length of the plant according to the present invention is 1,5 to 5 times longer, particularly 2 to 4 times longer, more particularly 2 to 3,5 times longer than the internode length of a cultivated *Cucurbita pepo* plant of the same type at the same ready-to-harvest stage, that does not contain the trait of elongated internodes which is obtainable from a plant grown from seed of *Cucurbita pepo* plant PARTYEL having been deposited the 2^{nd} of March 2011, under NCIMB Accession No. 41814.

The term "short petiole length" is herein understood as a length between about 35 cm and about 45 cm, between about 38 cm and 42cm, more preferably about 40 cm.

"Marketable fruits", as used herein, refer to non shrunken, non deformed fruits, wherein said fruits typically exhibit a blossom end diameter of between about 2.5 cm and about 7 cm, particularly between 3 and 5 cm, more particularly between 4 and 6 cm and have a length of between about 12 and about 30 cm, particularly between about 15 to about 25 cm, more particularly between about 16 and about 22 cm.

The marketable fruit "yield", as used herein, refer to the number of marketable fruits (mrktf) produced per plant (mrktf/p).

The "ready-to-harvest stage", as used herein, refers to the growth stage period of the plant where a fruit setting at a given node has reached its marketable size, as a marketable fruit as it has been defined previously.

### EMBODIMENTS

In embodiment 1, the invention discloses a cultivated *Cucurbita pepo* plant, which is capable of producing parthenocarpic fruits and exhibits elongated internode length, wherein the parthenocarpic trait is controlled by a genetic factor and is independent of pollination and parthenocarpy-inducing chemical treatments regimes, and wherein the elongated internode length trait is controlled by a genetic factor and is characterized in that the internode length is 1.5 to 5 times longer, particularly 2 to 4 times longer, more particularly 2 to 3.5 times longer than the internode length of a cultivated *Cucurbita pepo* plant of the same type at the same ready-to-harvest stage.

In embodiment 2, the invention discloses a plant according to embodiment 1, wherein said elongated internode length trait is characterized in that the internode length is comprised between 3.5 and 11 cm, preferably between 5 and 11 cm, but more preferably between 5 and 9 cm.

In embodiment 3, the invention discloses a plant according to any one of embodiments 1 to 2, wherein said parthenocarpic trait and said elongated internode length trait are obtainable from a donor plant which has the genetic background of *Cucurbita pepo* plant PARTYEL, representative seed of which is deposited under NCIMB Accession No. 41814.

In one embodiment, the genetic factor controlling the trait of parthenocarpy and the genetic factor controlling the trait of elongated internodes length can be found in the deposited seeds of plant PARTYEL having been deposited under NCIMB Accession No. 41814, and can be introduced into any other *Cucurbita pepo* plant by various means such as crossing, optionally followed by back-crossing, or by transformation techniques.

In one embodiment, the invention relates to *Cucurbita pepo* plant exhibiting trait of parthenocarpy and trait of elongated internodes, wherein the trait of parthenocarpy and the trait of elongated internodes length is obtainable by introgression from a plant grown from seed of *Cucurbita pepo* plant PARTYEL having been deposited under NCIMB Accession No. 41814.

In one embodiment, the invention relates to *Cucurbita pepo* plant exhibiting trait of parthenocarpy and trait of elongated internodes, wherein the trait of parthenocarpy and the trait of elongated internodes length are introgressed from a plant grown from seed of *Cucurbita pepo* plant PARTYEL having been deposited under NCIMB Accession No. 41814.

In embodiment 4, the invention discloses a plant according to any one of embodiments 1 to 3, wherein said plant is an inbred, a dihaploid or a hybrid.

In embodiment 5, the invention discloses a plant according to any of the embodiments 1 to 4, obtainable by crossing *Cucurbita pepo* plant PARTYEL, representative seed of which is deposited under NCIMB Accession No. 41814, with any *Cucurbita pepo* plant that does not contain at least one trait selected in the group comprising parthenocarpic trait and elongated internode length trait.

In embodiment 6, the invention discloses seed of a *Cucurbita pepo* plant according to any one of embodiments 1 to 5.

In embodiment 7, the invention discloses fruit of a *Cucurbita pepo* plant according to any one of embodiments 1 to 5.

In embodiment 8, the invention discloses the use of a *Cucurbita pepo* cultivation material, seed or plant, comprising genetic factors contributing to parthenocarpic trait and elongated internode length trait for cultivation on a support in a protected area for production and harvesting of fruits, independently of any chemical treatment and/or without any pollination.

In embodiment 9, the invention discloses the use of a cultivated *Cucurbita pepo* plant material according to embodiment 8, wherein said material is a material according to any one of embodiments 1 to 6.

In embodiment 10, the invention discloses the use of a cultivated *Cucurbita pepo* plant, or plant part, according to any one of embodiments 1 to 6 to produce a hybrid *Cucurbita pepo* plant suitable for cultivation on a support in a protecting area for production and harvest of fruits.

In embodiment 11, the invention discloses a method for producing *Cucurbita pepo* fruits from a *Cucurbita pepo* plant without pollination, nor chemical treatment, wherein said method comprises steps of:
a) providing a *Cucurbita pepo* cultivation material, seed or plant, wherein said material is a material according to any one of embodiments 1 to 6,
b) growing the material provided in a protected area and,
c) directing the growth of the plant along a support or trellis until fruit setting, and,
d) harvesting the fruits once they reach the ready-to-harvest stage.

In embodiment 12, the invention discloses a method of providing a *Cucurbita pepo* material, seed or plant, wherein said method comprises the following steps:
a) Providing a 1^{st} *Cucurbita pepo* plant,
b) Crossing said 1^{st} plant with a 2^{nd} *Cucurbita pepo* plant comprising a "parthenocarpic" trait and a "elongated internode length" trait,
c) Obtaining a progeny *Cucurbita pepo* plant and,
d) Selecting a plant of said progeny characterized in that said plant produces parthenocarpic fruits in a protected area without pollination nor chemical treatment, and wherein said plant is characterized by an elongated internode length.

In embodiment 13, the invention discloses a method according to embodiment 12, wherein the 2^{nd} *Cucurbita pepo* plant is chosen in the group comprising plant according to any one of embodiments 1 to 5.

In embodiment 14, the invention discloses a cell of a plant according to any one of embodiments 1 to 5.

In a further embodiment 15, the invention discloses a plant according to any one of embodiments 1 to 5, wherein said plant is capable of producing between about 1700 g and about 2500 g of marketable fruits, more preferably between about 2000 g and about 2500 g of marketable fruits.

In a further embodiment 16, the invention discloses a plant according to any one of embodiments 1 to 5, wherein said plant is capable of producing between 9 and 30 marketable fruits, preferably between 17 and 25 marketable fruits, more preferably between 20 and 25 marketable fruits.

In a further embodiment 17, the invention discloses a plant according to any one of embodiments 1 to 5, wherein said plant provides fruits having a blossom end diameter of between about 2.5 cm and about 7 cm, particularly between 3 and 5 cm, more particularly between 4 and 6 cm, and fruit length of between about 12 and about 30, particularly between about 15 to about 25 cm, more particularly between about 16 and about 22 cm.

In a further embodiment 18, the invention discloses a plant according to any one of embodiments 1 to 5, wherein said plant displays petioles having a length of between about 35 cm and about 45 cm, more preferably displays petioles having a length of about 40 cm.

In a further embodiment 19, the invention discloses a plant having UPOV characteristics according to Table 1 further, wherein said plant produces parthenocarpic fruits.

In a further embodiment 20, the invention discloses a method according to embodiment 11, wherein the *Cucurbita pepo* cultivation material, seed or plant, provided for step a) of said method is characterized by its capability of producing between about 1700 g and about 2500 g of marketable fruits, more preferably between about 2000 g and about 2500 g of marketable fruits.

In a further embodiment 21, the invention discloses a method according to embodiment 11, wherein the *Cucurbita pepo* cultivation material, seed or plant, provided for step a) of said method is characterized by its capability of producing between 9 and 30 marketable fruits, preferably between 17 and 25 marketable fruits, more preferably between 20 and 25 marketable fruits.

In a further embodiment 22, the invention discloses a method according to embodiment 11, wherein the *Cucurbita pepo* cultivation material, seed or plant, provided for step a) of said method is characterized by its capability of providing fruits having a blossom end diameter of between about 2.5 cm and about 7 cm, particularly between 3 and 5 cm, more particularly between 4 and 6 cm, and fruit length of between about 12 and about 30, particularly between about 15 to about 25 cm, more particularly between about 16 and about 22 cm.

In a further embodiment 23, the invention discloses a method according to embodiment 11, wherein the *Cucurbita pepo* cultivation material, seed or plant, provided for step a) of said method is characterized by its phenotype consisting of displaying petioles having a length of between about 35 cm and about 45 cm, more preferably displays petioles having a length of about 40 cm.

In a further embodiment 24, the invention discloses a method according to embodiment 12, wherein the *Cucurbita pepo* plant progeny selected within the step d) of said method is characterized by its capability of producing between about 1700 g and about 2500 g of marketable fruits, more preferably between about 2000 g and about 2500 g of marketable fruits.

In a further embodiment 25, the invention discloses a method according to embodiment 12, wherein the *Cucurbita pepo* plant progeny selected within the step d) of said method is characterized by its capability of producing between 9 and 30 marketable fruits, preferably between 17 and 25 marketable fruits, more preferably between 20 and 25 marketable fruits.

In a further embodiment 26, the invention discloses a method according to embodiment 12, wherein the *Cucurbita pepo* plant progeny selected within the step d) of said method is characterized by its capability of providing fruits having a blossom end diameter of between about 2.5 cm and about 7 cm, particularly between 3 and 5 cm, more particularly between 4 and 6 cm, and fruit length of between about 12 and about 30, particularly between about 15 to about 25 cm, more particularly between about 16 and about 22 cm.

In a further embodiment 27, the invention discloses a method according to embodiment 12, wherein the *Cucurbita pepo* plant progeny selected within the step d) of said method is characterized by its phenotype consisting of displaying petioles having a length of between about 35 cm and about 45 cm, more preferably displays petioles having a length of about 40 cm.

In a further embodiment 28, the invention discloses a use of a cultivated *Cucurbita pepo* material according to embodiments 8 to 10, wherein said cultivated *Cucurbita pepo* material is characterized by its capability of producing between about 1700 g and about 2500 g of marketable fruits, more preferably between about 2000 g and about 2500 g of marketable fruits.

In a further embodiment 29, the invention discloses a use of a cultivated *Cucurbita pepo* material according to embodiments 8 to 10, wherein said cultivated *Cucurbita pepo* material is characterized by its capability of producing between 9 and 30 marketable fruits, preferably between 17 and 25 marketable fruits, more preferably between 20 and 25 marketable fruits.

In a further embodiment 30, the invention discloses a use of a cultivated *Cucurbita pepo* material according to embodiments 8 to 10, wherein said cultivated *Cucurbita pepo* material is characterized by its capability of providing fruits having a blossom end diameter of between about 2.5 cm and about 7 cm, particularly between 3 and 5 cm, more particularly between 4 and 6 cm, and fruit length of between about 12 and about 30, particularly between about 15 to about 25 cm, more particularly between about 16 and about 22 cm.

In a further embodiment 31, the invention discloses a use of a cultivated *Cucurbita pepo* material according to embodiments 8 to 10, wherein said cultivated *Cucurbita pepo* material is characterized by its phenotype consisting of displaying petioles having a length of between about 35 cm and about 45 cm, more preferably displays petioles having a length of about 40 cm.

### BRIEF DESCRIPTION OF THE FIGURES

Fig. 1 is a schematic representation of the pedigree of PARTYEL plant, the numbers in red represent new material ID resulting from crosses or generations;
Fig. 2 is a graphic representation of cumulative marketable yield over period of Time in weeks, in PARTYEL plant (◆) as compared to NAA-treated CORA control plants (+) and untreated CORA control plants (▲). The cumulative marketable yield was measured over an 8-weeks time period. The evolution of the minimal temperature (□) observed in Gedera during this period is also showed.
Fig. 3 is a schematic representation of a method for determination of marketable fruit size;
Fig. 4 is a schematic representation of a method of measuring leaf petiole length;
Fig. 5 is a schematic representation of a measurement method of internodes length;
Fig. 6 is a diagram illustrating the distribution of genotypes according to yield categories indicating % marketable fruits/plant in 4 investigated crosses. D = PARTYEL plant and L1-4 are parental lines with corresponding F1 and F2 populations;
Fig. 7 is a diagram illustrating the distribution of genotypes according to leaf petiole length categories for the same crosses as for fig. 6;

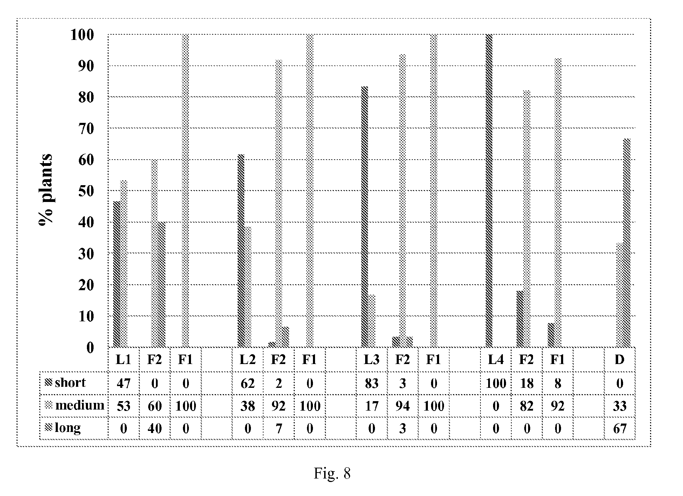

Fig. 8 is a diagram illustrating the distribution of genotypes according to internodes length categories for the same crosses as for fig. 6.

### Method for producing a New Summer Squash with a Phenotype for Protected Cultivation

In general terms the breeding procedure used herein comprises the steps of: (i) crossing two inbred lines or hybrid varieties to produce an F1 generation (ii) and self pollinate the F1 generation plants to produce the F2 generation that segregates for all factors for which the inbred parents differ. Variations of this generalized method are used, but all these variations produce a segregating generation which contains a range of variation for the characteristics of interest.

The described pedigree breeding approach was used to create a novel summer squash plant phenotype, exemplified by plant PARTYEL.

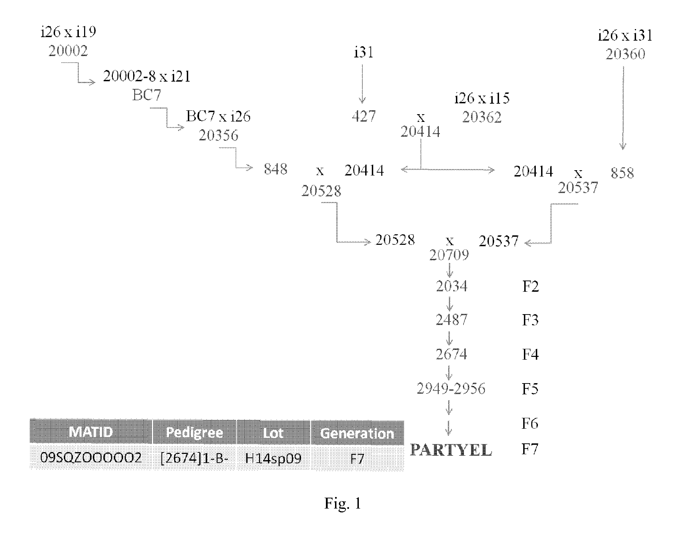

The initial genetic pool was created by crossing and self pollination of 5 commercial cultivars of summer squash designated as follows:126,119, 121,115, and 131 (Fig.1).

The initial 3 crosses (126 x 119) (126 x 115) (126 x 131) were made during 2003. In spring 2004, F2 population was generated from each cross. That F2 population was exposed to selection in isolated greenhouse during autumn 2004. The selection was made for plants that set a high proportion of marketable quality fruits, combined with short petioles and longer internodes. The selected plants were self pollinated and backcrossed again to cultivar 121 to enhance fruit desirable characteristics, and to 126 to enhance the expression of plant desirable characteristics (petioles and internodes lengths). New F2 populations were generated from new backcrosses in spring 2005. New population of F2 plants were exposed to same selection criterions during the 2005 autumn season. The selected individuals were self pollinated and F3 generations were tested during autumn 2006. The F3 family designated as 2487 was selected to present the ideal type for the novel squash plant type designed for greenhouse, i.e. parthenocarpy and elongated internode length as well as short petioles length. During 2007 the individual plants selected from 2487 underwent several self pollination till F7 and plant PARTYEL was selected for its properties of high degree of parthenocarpy, elongated internode length as well as short petiole length. PARTYEL is a squash plant representative of the *Cucurbita pepo* plant according to the present invention. Representative seeds of plant PARTYEL were deposited at NCIMB the 2^{nd} of March 2011 under NCIMB Accession No. 41814.

According to the present invention, a squash plant is provided having the characteristics of setting high proportion of parthenocarpic marketable fruits, under protected cultivation conditions (without pollination nor chemical treatment), combined with short petioles and elongated internodes lengths.

### Squash plant PARTYEL with improved parthenocarpic fruit set ability:

Summer squash plant PARTYEL has been developed to perform genetic parthenocarpy in an insect protected greenhouse.

The plants PARTYEL exhibits genetic ability to set parthenocarpic marketable fruits without inducing external chemical growth regulators such as Auxins or any other known parthenocarpy-inducing chemical regimes.

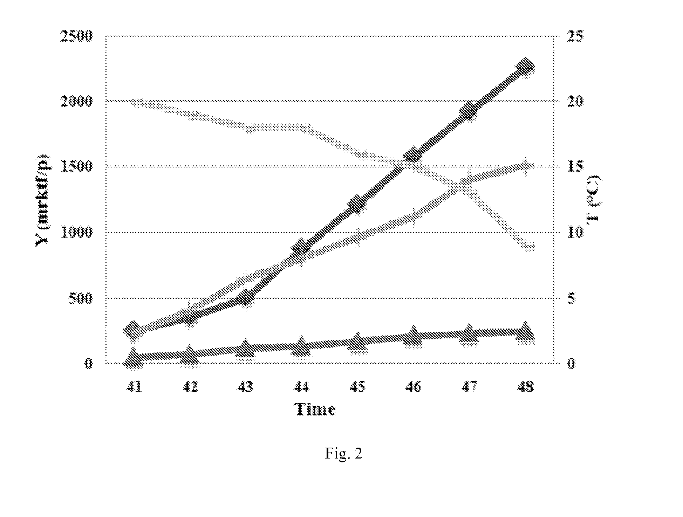

The parthenocarpy of PARTYEL was evaluated during fall 2008 in Gedera, Israel (Fig. 2). The cumulative yield pattern of plant PARTYEL was compared to CORA control varieties, over a period of 8 weeks of harvest from the first week of October to the last week of November.

Control varieties achieved a yield of only 254 grams/plant.

Control plants treated with growth regulator NAA (1- naphthalene acetic acid, Sodium salt) (Mancini et.al and Suleiman et.al.) yielded 1500 grams per plant. These findings support the knowledge that growth regulators application is essential to raise marketable yield in conventional squash varieties.

PARTYEL plant obtained a yield of 2259 grams per plant without growth regulators application, indicating presence of genetic determination that enhances spontaneous parthenocarpy fruit set and growth without pollination or chemicals induction as compared to standard squash varieties.

### Squash plant PARTYEL with plant habit adapted for protected cultivation.

The PARTYEL plant characteristics were described following UPOV guidelines (Table 1).

**Table 1: Description of plant PARTYEL according to UPOV guidelines for squash**

| *Group* | *Characteristic* | *PARTYEL* |
|---|---|---|
| Plant | growth habit | trailing |
| | Branching | medium |
| Stem | Colour | green |
| | intensity of green colour | medium |
| | Mottling | absent |
| | Tendrils | absent |
| Leaf blade | Size | medium-small |
| | Incisions | deep |
| | intensity of green colour of upper surface | medium |
| | silvery patches | absent |
| | relative area covered by silvery patches | small |
| Petiole | Length | short |
| | number of prickles | medium |
| Female flower | ring at inner corolla | absent |
| Male flower | ring at inner corolla | absent |
| Young Fruit | length/ max diameter ratio | medium |
| | general shape | tapered cylindrical |
| | main color of skin | yellow |
| Fruit | general shape | tapered cylindrical |
| | Neck | absent |
| | Groves | absent |
| | Ribs | absent |
| | main color of skin | light yellow |
| | Dots | absent |
| | Warts | absent |
| | size of flower scar | very small |
| | length of peduncle | long |
| | color of peduncle | partly yellow partly green |
| | intensity of green color of peduncle | light |
| | mottling of peduncle | absent |
| Ripe fruit | main color of skin | Yellow |
| | intensity of main skin color | Light |
| Seeds | seed size | Small |

Two main characteristics referring to plant phenotype were identified to have positive effect on plant performance and adaptability to protected conditions: 1. Short petioles, 2. longer internodes.

The plant PARTYEL exhibits maximal petiole length of 40 cm.

The plant PARTYEL exhibits maximal internodes length of 9 cm.

Reference is now made to Table 2, presenting leaf petiole length and internodes length of the plant stem measured according to method disclosed in Examples 2 and 3 below.

**Table 2: Dimensions of petiole and internodes in PARTYEL compared to control cultivars.**

| **ZG Code** | **Designation** | **Petiole length (cm)** | **Internode length (cm)** |
|---|---|---|---|
| PARTYEL | D | 40±2 | 9.2±2.5 |
| Cora F1 | C1 | 66±6 | 2.1±0.5 |
| Natura F1 | C2 | 67±5 | 2.5±0.5 |

As shown in Table 2, PARTYEL exhibited significantly shorter mature leaf petioles and longer internodes length compared to control varieties Cora and Natura.

### EXAMPLE 1

### Method of growing summer squash plants in protected greenhouse:

The development of a new summer squash plant designed for protected cultivation requirements involves the following steps, procedures and regimes:
Plant in greenhouse covered with insect proof nets plastic or glass to prevent bees or other natural pollination insect activity.
   - No hormones or chemical treatments regime for inducing artificial fruit set.
   - Spacing of plants between the rows 180- 210 cm and within the rows 40-50 cm depends on location.
   - The plants are irrigated and fertilized using pipe irrigation system as practiced in the area
   - The plants are trellised and supported vertically by a single stem on a wire.
   - Side shots on the first 3-4 internodes should be pruned.

### EXAMPLE 2

### Method of measuring a parthenocarpic fruit set in protected and pollination preventing conditions

The most accurate practice to measure the yield in summer squash is by counting number of marketable size fruits per plant over harvesting period of at least 8 weeks.

The following practice was applied to measure the parthenocarpy trait in summer squash plant as presented:
- After planting and establishment in greenhouse of young premature plants, each plant is labeled on the wire by serial number (1,2,3...) within each plot and row.
- After first female flowering begins, tag every female flower in the trial, indicating day of anthesis by calendar date.

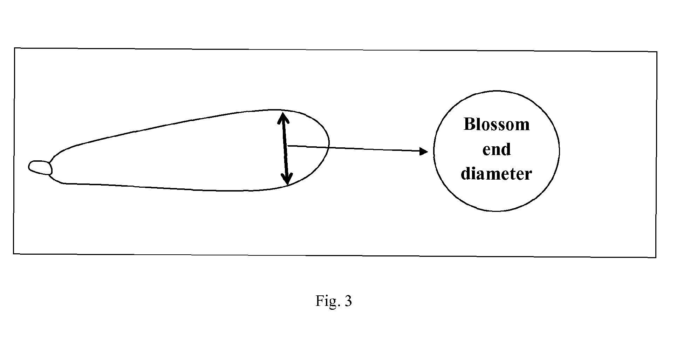

Fruit development is checked every day using 4 cm ring caliber indicating the general marketable diameter for grade 1 fruits. Fruit reaching 4 cm at blossom end is harvested with the tag indicating the anthesis day, plot and plant serial number (fig. 3). The desired fruit length is between about 16 cm and about 22 cm and the desired fruit diameter is between about 2.5 cm and about 4 cm.

### EXAMPLE 3

### Method of measuring plant habit related traits

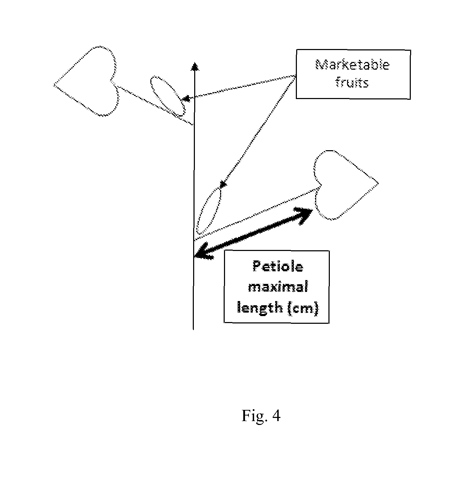

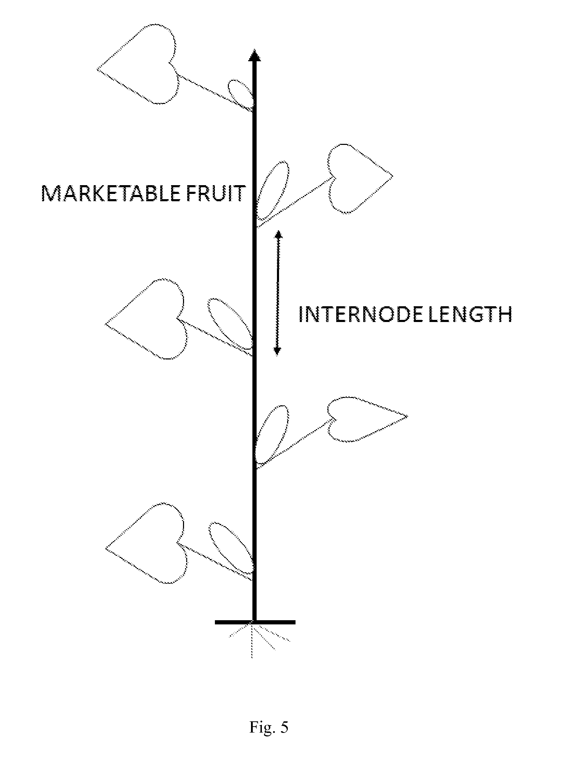

Additional data from each plant in the trial is taken from the middle part of mature plant at ready-to-harvest stage (70-80 days after planting):
1. Petiole length at fully expanded mature leaf (Fig. 4).
2. Internode length underneath a marketable fruit (Fig. 5).

### EXAMPLE 4

### Example of breeding new summer squash cultivar designed for protected conditions using PARTYEL.

Squash plants genetic breeding includes both self-pollination and cross-pollination techniques. The presented example was conducted during fall 2009, and it provides solid evidence that marketable fruit yield and plant habit related traits as detailed above, are genetically inherited in two separate environments (Israel and Spain). Particularly, it is herein shown that the trait of parthenocarpy as contained in PARTYEL can be transferred and bestow within any squash plant by traditional crossing, including back-crossing to produce a hybrid progeny containing the trait, for example. Furthermore the trait of elongated internode length is also shown to be transferable from PARTYEL to any other squash plant material by crossing, including back-crossing in order to produce progeny plant containing that trait as well for example. Both traits being independently inherited, a man skilled in the art can easily check and select the plant of the progeny that contain the traits of interest according to the present invention by simple measurements.

PARTYEL Plant was crossed with 4 summer squash parental lines of cultivated plants (designated as L1-4) that do not contain the trait of parthenocarpy and do not contain the trait of elongated internode length to produce F1 hybrids. The lines were selected to represent different commercial types of summer squash plants from white zucchini to dark green zucchini. Those squash lines to be crossed with plant PARTYEL are all bushy type plants and correspond to parental lines of classically commercially available squash hybrid plants. Any type of squash plant, particularly commercial squash plant may be used for crossing with plant PARTYEL in order to transfer the traits of parthenocarpy and elongated internodes length to the progeny. Two commercial cultivars were added as controls to the experiment (Table 3).

| Table 3: Description of summer squash plant lines and control cultivars which participated in the example, according to their commercial classification. | | | |
|---|---|---|---|
| ZG Code | Seeds origin | Designation | Type |
| PARTYEL | Zeraim Gedera | D | Yellow Zucchini |
| 2943 | Zeraim Gedera | L1 | White Zucchini |
| 2667 | Zeraim Gedera | L2 | Medium Green Zucchini |
| 3146 | Syngenta | L3 | Medium Green Zucchini |
| 3145 | Syngenta | L4 | Dark Green Zucchini |
| Cora F1 | Claude Tesier | C1 | Medium Green Zucchini |
| Natura F1 | Enza Zaden | C2 | Medium Green Zucchini |

The F1 hybrids were self pollinated to generate F2 populations.

All plants in the trial were transplanted under plastic covered and insects protected unheated greenhouse in Almeria, Spain on 10-Sep-2009. An additional trial was planted in the same way in Gedera, Israel on 15-Sep-2009.

The parental lines plants and control plants were replicated randomly to estimate environmental variation. The ANOVA analysis variation for measured traits shows that the genotype effect was significant in both locations. The environmental effect brought about by the location of the plant in the greenhouse was very low or negligible compare to the genetic effect (Table 5).

| Table 5: ANOVA variance analysis. Effects on experimental variation in Spain and Israel | | | | | | | |
|---|---|---|---|---|---|---|---|
| | | **Spain** | | | **Israel** | | |
| **Category** | **Source of variation** | **'d.f.'** | **'F'** | **'Prob>F'** | **'d.f.'** | **'F'** | **'Prob>F'** |
| **Number of normal fruits** | **Genotype** | **14** | **17.458** | **0.000** | **14** | **23.146** | **0.000** |
| | **Enviroment** | **16** | **1.633** | **0.058** | **11** | **1.886** | **0.039** |
| **Percentage of normal fruits** | **Genotype** | **14** | **13.140** | **0.000** | **14** | **10.678** | **0.000** |
| | **Enviroment** | **16** | **1.592** | **0,068** | **11** | **1.484** | **0.134** |
| **Internode length** | **Genotype** | **14** | **40.869** | **0.000** | **14** | **9.779** | **0.000** |
| | **Enviroment** | **16** | **1,763** | **0.034** | **7** | **1.348** | **0.228** |
| **Petiole length** | **Genotype** | **14** | **29.032** | **0.000** | **14** | **19.384** | **0.000** |
| | **Enviroment** | **16** | **1.105** | **0,348** | **11** | **0.927** | **0.514** |

### 1. Parthenocarpy assessment:

Marketable fruit yield was measured from each individual plant, during 8 weeks of harvest from first week of October (week 41) until last week of November (week 48). The individual plants from all genotypes were clustered into three yield categories of marketable fruits/plant (mrktf/p): the low yield category (0-8 mrktf/p), the medium yield category (9-16 mrktf/p) and the high yield category (17-25 mrktf/p).

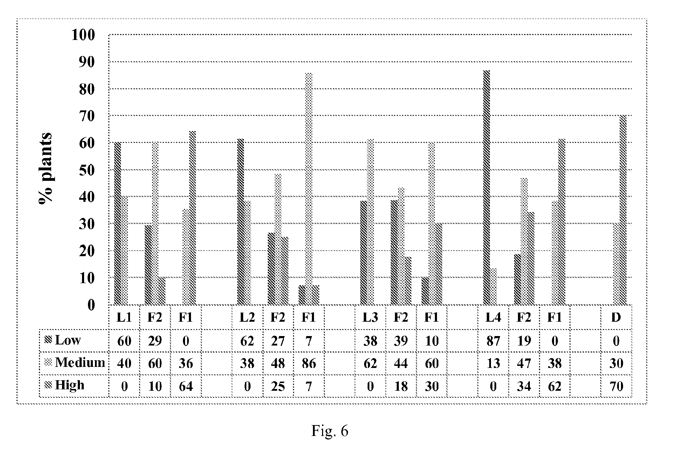

Analysis of the partitioning of plants into proposed yield categories (Fig. 6) clearly indicates the presence of F2 individual plants that completely fit the low and high yield categories.

For all four different control lines, L1-4, there is no plant in the high yield category, but on the other hand, 70% of PARTYEL plants produce a high yield. The proportion of F2 individuals that produced a high yield, similar to the donor plant PARTYEL, varied from 10% to 34% depending upon specific crosses. In fact, the high yield plants presented yield much higher compared to what had been reported previously (Robinson *et al.*; Nijs *et al.*). The higher yield plants reached more than 20 marketable fruits/ plant and over 2000 grams/plant compare to 4 fruits and 1000 grams been reported by Nijs *et.al,* 1982.

F1 population over-performed the non parthenocarpic parental lines L1-4 indicating that parthenocarpic trait originated in PARTYEL is inherited in incomplete dominance mode. The distribution of F2 individuals across yield categories indicates additive manner of inheritance in all presented crosses.

### 2. Petiole length assessment:

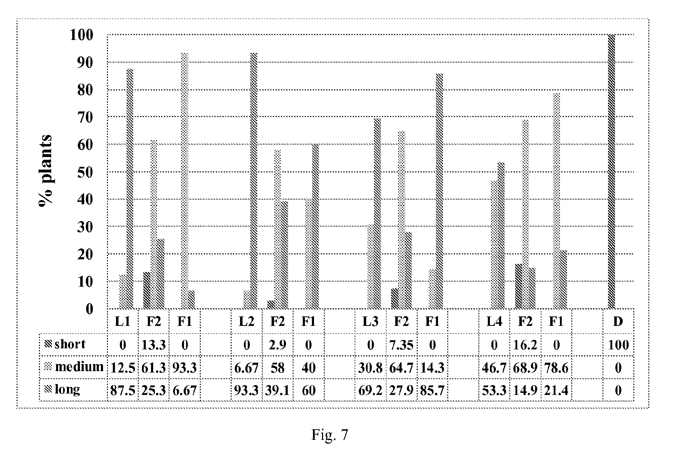

The petiole length of mature leaves in the experiments varied from 35 to 65 cm. The distribution of plants into categories of petiole length is presented in Fig. 7. PARTYEL had uniformly short petiole in short category (35-45 cm), compared to parental lines (L) that varied within medium (45 - 55 cm) and long category (56-65 cm).

F2 populations segregated to all 3 categories with clear dominant effect toward longer petioles. The proportion of F2 plants in short category varied from 3% in cross PARTYELxL2 to 16% in cross PARTYELxL4 indicating that the short petiole trait is inheritable and indeed inherited.

### 3. Internode length assessment:

Length of four consequent internodes was measured in the plant at ready-to-harvest stage as described in Example 3. The average single internode length varied from 1.5 cm (very short) to 11 cm (very long) internode length. This length range was divided into 3 length categories. The parental lines (L) exhibited short internode length in the range of 1.5 to 3.2 cm while PARTYEL varied from 8-11 cm. The medium category was determined as 3.5-7.5 cm.

The result indicates that the elongated internode length trait is dependent upon the specific cross. For example in cross PARTYELxL1 the proportion of F2 plants in the long category was 40% whereas in cross PARTYELxL4 the proportion in that category was 0%. The strong additive effect with dominance toward longer internodes, concentrates the F1 and F2 population into medium length category. On the other hand medium length internodes may fit the optimal internodes length desired for well balanced summer squash plant adapted to vertical trellising as practiced in protected cultivation. Breeding of such phenotypes of hybrid cultivars are achieved by crosses between lines with opposite phenotypes. In any case, whatever the internodes length of the parent that is crossed with the PARTYEL plant, the internodes length of the progeny is increased as compared to the one of the parent plant that does not contain the elongated internodes length trait, showing that that trait is inheritable from that donor plant and that it can be bestowed in any other genetic background.

### 4. Conclusions

The role of stable genetically controlled parthenocarpy is critical for successful breeding of future summer squash varieties for protected cultivation. Plant structure related traits such as short leaf petioles and elongated internode length were identified to play positive role to increase the effectiveness of greenhouse space usage in summer squash production and increase yield and easiness of harvesting.

The core of the present invention, as exemplified by the novel summer squash plant PARTYEL provides the desired combination of stable parthenocarpy combined with plant habit related traits that contribute to a useful product. The plant habit reduces the incidence of disease, and reduces the need to use chemical sprays. The plants can be grown in higher density than in the usual practice. Thereby this combination of traits contributes to the effectiveness of space usage in greenhouses by increasing the number of plants per square meter that can be grown. The core of the present invention also includes within its scope *Cucurbita pepo* plants that fit the special climatic and technological conditions characterizing protected cultivation (Luzny. J.), especially by providing plants of a smaller volume phenotype better adapted to forced conditions in greenhouses. A precise method of growing summer squash plants in protected conditions is essential for effective breeding. The appropriate methods were developed to grow and measure the phenotypes. The plants are grown in a protected area under the appropriate conditions of irrigation and fertilization, and the growth of the plant is directed along a support or trellis until fruit setting. The fruits are harvested once they reach a ready-to-harvest stage. The phenotypic performances highlighted within these experiments are obtained under protected conditions or in a protected area, more particularly in passive green houses in Almeria (Spain) and in Gedera (Israel) during autumn season from beginning of September to end of November. The table bellow shows average outside temperatures and mid-month day light length for the particular area.

| Season | Autumn | | | | | |
|---|---|---|---|---|---|---|
| Location | Almeria, Spain | | | Gedera, Israel | | |
| Months | Sep. | Oct. | Nov. | Sep. | Oct. | Nov. |
| Max mean temperature C | 28.9 | 23.9 | 19.3 | 30.4 | 28.3 | 24.1 |
| Min mean temperature C | 20.2 | 15.9 | 11.6 | 19.4 | 16 | 11.8 |
| Day length(hours, minutes) | 12h 25m | 11h 17m | 10h 15m | 12h 22m | 11h 25m | 10h 32m |

The presented novel example shows that the described combination of traits as contained in plant PARTYEL are genetically controlled and independently inherited. Those genetic factors contribute to the phenotype of parthenocarpy, elongated internode length as well as short petiole length. Thus, selection and breeding of *Cucurbita pepo* plants bearing such traits are now made possible thanks to the present invention. By analyzing the F2 population it was possible to identify individual plants that combine the desired traits of parthenocarpy, elongated internode length and short petioles length in an average frequency of 3% in all crosses presented in the examples.

The foregoing invention has been described in detail by way of illustration and example for purposes of clarity and understanding. However, it will be obvious that certain changes and modifications such as single gene modifications and mutations, somaclonal variants, variant individuals selected from large populations of the plants of the instant inbred and the like may be practiced within the scope of the invention, as limited only by the scope of the appended claims.

### Bibliography

- Robinson, R.W. and Reiners, S. (1999). Parthenocarpy in summer squash. HortScience 34: 715-717.
- US Patent 6031158 - Parthenocarpic trait in summer squash
- Mancini, L., Calabrese, N. Effect of growth regulators on flower differentiation and yield in zucchini (Cucurbita pepo L.) grown in protected cultivation Acta Horticulturae. 1999. 492, 265-272.
- United States Standards for Grades of Summer Squash
- Suleiman, F. A. S.; Suwwan, M. A. Effect of agritone on fruit set and productivity of summer squash (Cucurbita pepo L.) under plastic house conditions. Advances in Horticultural Science 4(1990): 83-89
- A.P.M. den Nijs, and N.J.D. Veldhuyzen van Zanten. Parthenocarpic fruit set in glasshouse grown zucchini squash. Cucurbit Genet. Coop.Rpt. 5:44-45. 1982.
- A.P. M. den Nijs, and J. Balder. Growth of Parthenocarpic and Seed-bearing Fruit of Zucchini Squash Cucurbit Genetics Cooperative Report 6:84-85 (article 42) 1983
- J. Luzny. CONTRIBUTION TO BREEDING OF VEGETABLE CULTIVARS IN FORCING HOUSES ISHS Acta Horticulturae 17: Symposium on Protected Growing of Vegetables
- UPOV description guidelines for squash.

## Claims

1. A cultivated *Cucurbita pepo* plant, which is capable of producing parthenocarpic fruits and exhibits elongated internode length, wherein the parthenocarpic trait is controlled by a genetic factor and is independent of pollination and parthenocarpy-inducing chemical treatments regimes, and wherein the elongated internode length trait is controlled by a genetic factor and is **characterized in that** the internode length is 1,5 to 5 times longer, particularly 2 to 4 times longer, more particularly 2 to 3,5 times longer than the internode length of a cultivated *Cucurbita pepo* plant of the same type at the same ready-to-harvest stage.

2. A plant according to embodiment 1, wherein said elongated internode length trait is **characterized in that** the internode length is comprised between 3.5 and 11 cm, preferably between 5 and 11 cm, but more preferably between 5 and 9 cm.

3. A plant according to any one of embodiments 1 to 2, wherein said parthenocarpic trait and said elongated internode length trait are obtainable from a donor plant which has the genetic background of *Cucurbita pepo* plant PARTYEL, representative seed of which is deposited under NCIMB Accession No. 41814.

4. A plant according to any one of embodiments 1 to 3, wherein said plant is an inbred, a dihaploid or a hybrid.

5. A plant according to any of the embodiments 1 to 4, obtainable by crossing *Cucurbita pepo* plant PARTYEL, representative seed of which is deposited under NCIMB Accession No. 41814, with any *Cucurbita pepo* plant that does not contain at least one trait selected in the group comprising parthenocarpic trait and elongated internode length trait.

6. Seed of a *Cucurbita pepo* plant according to any one of embodiments 1 to 5.

7. Fruit of a *Cucurbita pepo* plant according to any one of embodiments 1 to 5.

8. Use of a *Cucurbita pepo* cultivation material, seed or plant, comprising genetic factors contributing to parthenocarpic trait and elongated internode length trait for cultivation on a support in a protected area for production and harvesting of fruits, independently of any chemical treatment and/or without any pollination.

9. Use of a cultivated *Cucurbita pepo* plant material according to embodiment 8, wherein said material is a material according to any one of embodiments 1 to 6.

10. Use of a cultivated *Cucurbita pepo* plant, or plant part, according to any one of embodiments 1 to 6 to produce a hybrid *Cucurbita pepo* plant suitable for cultivation on a support in a protecting area for production and harvest of fruits.

11. A method for producing *Cucurbita pepo* fruits from a *Cucurbita pepo* plant without pollination nor chemical treatment, wherein said method comprises steps of:
a. providing a *Cucurbita pepo* cultivation material, seed or plant, wherein said material is a material according to any one of claims 1 to 6,
b. growing the material provided in a protected area and,
c. directing the growth of the plant along a support or trellis until fruit setting, and,
d. harvesting the fruits once they reach the ready-to-harvest stage.

12. A method of providing a *Cucurbita pepo* material, seed or plant, wherein said method comprises the following steps:
a. Providing a 1^{st} *Cucurbita pepo* plant,
b. Crossing said 1^{st} plant with a 2^{nd} *Cucurbita pepo* plant comprising a "parthenocarpic" trait and a "elongated internode length" trait,
c. Obtaining a progeny *Cucurbita pepo* plant and,
d. Selecting a plant of said progeny **characterized in that** said plant produces parthenocarpic fruits in a protected area without pollination nor chemical treatment, and wherein said plant is **characterized by** an elongated internode length.

13. In embodiment 13, the invention discloses a method according to embodiment 12, wherein the 2^{nd} *Cucurbita pepo* plant is chosen in the group comprising plant according to any one of embodiments 1 to 5.

14. In embodiment 14, the invention discloses a cell of a plant according to any one of embodiments 1 to 5.
